# EUROPÄISCHE PATENTSCHRIFT

(11) **EP 3 861 952 B1**
(45) Veröffentlichungstag und Bekanntmachung des Hinweises auf die Patenterteilung: **09.10.2024**
(21) Anmeldenummer: 21151962.4
(22) Anmeldetag: 17.01.2021
(51) Int. Cl.: A61B 18/12, A61B 18/14, A61B 90/98, A61B 18/00, A61B 17/32, A61B 90/00

(54) **ELEKTROCHIRURGISCHES SYSTEM, ELEKTROCHIRURGISCHES INSTRUMENT, VERFAHREN ZUM SCHREIBEN VON BETRIEBSDATEN, UND ELEKTROCHIRURGISCHES VERSORGUNGSGERÄT**
ELECTROSURGICAL SYSTEM, ELECTROSURGICAL INSTRUMENT, OPERATING DATA WRITING METHOD, AND ELECTROSURGICAL POWER SUPPLY APPARATUS
SYSTÈME ÉLECTROCHIRUGICAL, INSTRUMENT ÉLECTROCHIRUGICAL, PROCÉDÉ D'ÉCRITURE DES DONNÉES DE FONCTIONNEMENT ET APPAREIL D'ALIMENTATION ÉLECTROCHIRUGICAL

(30) Priorität: 10.02.2020 DE 102020103278
(43) Veröffentlichungstag der Anmeldung: 11.08.2021
(73) Patentinhaber: OLYMPUS WINTER & IBE GMBH, 22045 Hamburg (DE)
(72) Erfinder: Kersten, Lutz, 12163 Berlin (DE); Kwik, Anne, 14165 Berlin (DE)
(74) Vertreter: Noack, Andreas

(56) Entgegenhaltungen:
- EP-A2- 0 363 863
- WO-A1-02/41798
- WO-A1-2011/000533
- DE-A1- 102016 108 081
- US-A1- 2004 044 641
- US-A1- 2009 248 007
- US-A1- 2013 046 292

## Beschreibung

Die Erfindung betrifft ein elektrochirurgisches System, umfassend: ein elektrochirurgisches Versorgungsgerät, und ein elektrochirurgisches Instrument, wobei das elektrochirurgische Instrument ein Speicherelement umfasst, welches durch das elektrochirurgische Versorgungsgerät ausgelesen und beschrieben werden kann, wenn das elektrochirurgische Instrument mit dem elektrochirurgischen Versorgungsgerät verbunden ist, und das elektrochirurgische Versorgungsgerät eingerichtet ist, Betriebsdaten auf das Speicherelement zu schreiben. Ferner betrifft die Erfindung ein Verfahren zum Schreiben von Betriebsdaten.

Elektrochirurgische Systeme werden in der Chirurgie seit längerem eingesetzt, um diverse Prozeduren durchzuführen. Bei der Elektrochirurgie im engeren Sinne wird zu behandelndes Gewebe direkt elektrischen Strömen ausgesetzt, bei denen es sich in der Regel um hochfrequente Wechselströme handelt. Durch entsprechende Dimensionierung der Instrumente und der verwendeten Ströme und Spannungen lassen sich unterschiedliche Gewebeeffekte erreichen, beispielsweise ein Koagulieren oder Schneiden des Gewebes. Es sind auch Systeme bekannt, bei welchen ein zu behandelndes Gewebe zusätzlich oder ausschließlich über eine Ultraschall-Sonotrode beaufschlagt wird. Hier befindet sich zumeist in dem anzuwendenden Instrument ein Transducer, welcher ein von einem Versorgungsgerät in Form eines Ultraschallgenerator bereitgestelltes elektrisches Signal in Ultraschallschwingungen der Sonotrode umwandelt. Solche Ultraschallsysteme werden im Sinne der Erfindung ebenfalls als elektrochirurgische Systems angesehen. Elektrochirurgische Instrumente weisen in der Regel nur eine begrenzte Lebensdauer auf. Dies gilt gleichermaßen für Instrumente, die ausschließlich für einen einmaligen Gebrauch spezifiziert sind, als auch für Instrumente, die für mehrmaligen Gebrauch bestimmt sind. Es wurden verschiedene Methoden entwickelt um sicherzustellen, dass ein elektrochirurgisches Instrument nicht über seine vorgesehene Anzahl von Verwendungen hinaus angewendet wird.

Bei modernen elektrochirurgischen Systemen ist dazu das Instrument mit einem Speicherelement ausgestattet, auf welchem Konfigurationsdaten für das Versorgungsgerät hinterlegt sind. Als ein Bestandteil der Konfigurationsdaten ist ein Betriebsdatenelement vorgesehen, in welches das elektrochirurgische Versorgungsgerät Betriebsdaten schreiben kann. Diese Betriebsdaten können Verbrauchsdaten sein, wie beispielsweise Anzahl und Dauer der Aktivierungen, abgegebene Energie/Leistung, Zeitstempel, Temperaturdaten oder ähnliches. Sobald ein elektrochirurgisches Instrument mit einem elektrochirurgischen Versorgungsgerät verbunden wird, kann das Versorgungsgerät das Betriebsdatenelement auslesen und bestimmen, ob eine weitere Verwendung des Instruments zulässig ist.

Gerade im Bereich der Elektrochirurgie kann es durch elektromagnetische Störungen dazu kommen, dass ein Schreibzugriff des elektrochirurgischen Versorgungsgeräts auf das Speicherelement im elektrochirurgischen Instrument fehlschlägt. In diesem Fall können die auf dem elektrochirurgischen Instrument gespeicherten Betriebsdaten kompromittiert sein, so dass nicht mehr verlässlich festgestellt werden kann, ob eine weitere Verwendung des elektrochirurgischen Instruments zulässig ist.

US-A-2013/046292 offenbart ein elektrochirurgisches System nach dem Oberbegriff des Anspruchs 1.

Es besteht daher eine Aufgabe der Erfindung darin, ein elektrochirurgisches System bereitzustellen, welches hinsichtlich der beschriebenen Problematik verbessert ist.

Diese Aufgabe wird gemäß der Erfindung gelöst durch ein elektrochirurgisches System nach Anspruch 1.

Durch die beschriebene Weiterbildung steht auch bei einem fehlgeschlagenen Schreibzugriff auf eines der Betriebsdatenelemente weiterhin das andere Betriebsdatenelement zur Verfügung, so dass die vorangehend dort abgespeicherten Betriebsdaten ausgelesen werden können. Somit kann der Nutzungszustand des elektrochirurgischen Instruments ggf. genau genug ermittelt werden, um über eine weitere Verwendung entscheiden zu können. In einer möglichen Weiterbildung der Erfindung kann die Datenstruktur ein Schreibflag umfassen, dessen Inhalt anzeigt, ob Betriebsdaten in das erste Betriebsdatenelement oder das zweite Betriebsdatenelement geschrieben werden sollen.

Dabei kann das elektrochirurgische Versorgungsgerät eingerichtet sein, nach dem erfolgreichen Schreiben von Betriebsdaten in das erste Betriebsdatenelement oder das zweite Betriebsdatenelement das Schreibflag zu toggeln.

In einer bevorzugten Ausführung eines elektrochirurgisches System nach der Erfindung kann in der Datenstruktur jedem der Betriebsdatenelemente ein Schreibflag zugeordnet sein, und das elektrochirurgische Versorgungsgerät kann eingerichtet sein, nach dem erfolgreichen Schreiben von Betriebsdaten in eines der Betriebsdatenelemente das diesem Betriebsdatenelement zugeordnete Schreibflag zu toggeln.

Dabei kann vorzugsweise das elektrochirurgische Versorgungsgerät eingerichtet sein, Betriebsdaten in das erste Betriebsdatenelement zu schreiben, wenn der Wert der Schreibflags ungleich ist, und Betriebsdaten in das zweite Betriebsdatenelement zu schreiben, wenn der Wert der Schreibflags gleich ist.

Die Aufgabe wird gemäß eines zweiten Aspekts der Erfindung gelöst durch ein Verfahren nach Anspruch 6.

Mittels des beschriebenen Verfahrens ist sichergestellt, dass immer nur auf eines der Betriebsdatenelemente schreibend zugegriffen wird. Bei einem Schreibfehler besteht somit eine hohe Wahrscheinlichkeit, dass das andere Betriebsdatenelement intakt bleib, und somit auf die zuletzt aktuellen Betriebsdaten zurückgegriffen werden kann.

In einer möglichen Weiterbildung eines Verfahren nach der Erfindung können die Werte von zwei Schreibflags ausgelesen werden, welche jeweils einem Betriebsdatenelement zugeordnet sind, wobei die Betriebsdaten in das erste Betriebsdatenelement geschrieben werden, wenn die Werte der beiden Schreibflags ungleich sind, und in das zweite Betriebsdatenelement geschrieben werden, wenn die Werte der beiden Schreibflags gleich sind.

Vorzugsweise kann nach dem Schreiben von Betriebsdaten in eines der Betriebsdatenelemente das diesem Betriebsdatenelement zugeordnete Schreibflag getoggelt werden.

Die Erfindung wird nachfolgend anhand einiger beispielhafter Darstellungen näher beschrieben, wobei die in den Darstellungen gezeigten Ausführungsbeispiele lediglich zum besseren Verständnis der Erfindung beitragen sollen, ohne diese einzuschränken.

Es zeigen:
- Fig. 1:: ein elektrochirurgisches System,
- Fig. 2:: eine Datenstruktur,
- Fig. 3:: einen Aufbau eines Satzes von Konfigurationsdaten,
- Fig. 4:: ein Verfahren zum Lesen und Schreiben von Betriebsdaten,
- Fig. 5:: ein Verfahren zum Auslesen von Konfigurationsdaten.

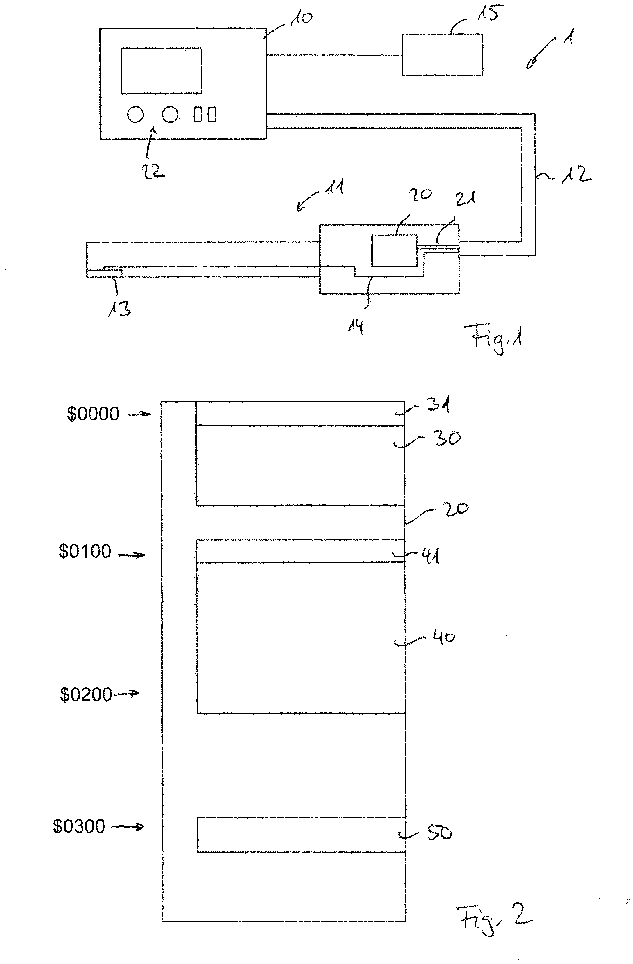

Figur 1 zeigt ein elektrochirurgisches System 1 mit einem elektrochirurgischen Versorgungsgerät 10 in Form eines Hochfrequenzgenerators, und mit einem elektrochirurgischen Instrument 11. Das elektrochirurgische Instrument 11 ist über ein Kabel 12 mit dem elektrochirurgischen Versorgungsgerät 10 verbunden. Anstelle des dargestellten Kabels 12 kann die Verbindung zwischen dem elektrochirurgischen Instrument 11 und dem Versorgungsgerät 10 auch kontaktlos, beispielsweise mit Hilfe von NFC (Near Field Communication) bzw. RFID (Radio Frequency Identification) hergestellt werden.

An einem distalen Ende des elektrochirurgischen Instruments 11 ist eine Elektrode 13 angeordnet, mit welcher Gewebe behandelt werden kann. Dazu ist die Elektrode 13 über eine Leitung 14 mit dem elektrochirurgischen Versorgungsgerät 10 verbunden.

Das elektrochirurgische Instrument kann mehr als eine Elektrode aufweisen. Alternativ oder zusätzlich zu der Elektrode 13 kann das elektrochirurgische Instrument 10 einen oder mehrere Ultraschall-Transducer umfassen.

Das elektrochirurgische Versorgungsgerät 10 erzeugt ein hochfrequentes elektrisches Signal, welches über die Leitung 14 an die Elektrode 13 geleitet wird und dort einen therapeutischen Effekt auf nicht dargestelltes Gewebe ausübt. Um den elektrochirurgischen Stromkreis zu schließen, kann eine Neutralelektrode 15 vorgesehen sein, welche ebenfalls mit dem elektrochirurgischen Versorgungsgerät 10 verbunden ist.

Das elektrochirurgische Instrument 11 ist mit einem Speicherelement 20 ausgestattet, auf welchem Konfigurationsdaten hinterlegt sind. Sobald das elektrochirurgische Instrument 11 mit dem elektrochirurgischen Versorgungsgerät 10 verbunden ist, liest das elektrochirurgische Versorgungsgerät 10 das Speicherelement 20 über Leitungen 21 aus. Das elektrochirurgische Versorgungsgerät 10 nutzt die von dem Speicherelement 20 geladenen Konfigurationsdaten, um das elektrische Signal einzustellen, welches an das elektrochirurgische Instrument 11 abgegeben wird. Dabei können verschiedene Eigenschaften des elektrischen Signals auch über Kontrollelemente 22 an dem elektrochirurgischen Versorgungsgerät 10 beeinflusst werden.

Die Konfigurationsdaten sind auf dem Speicherelement 20 in einer flexiblen Datenstruktur abgelegt, die in Figur 2 schematisch dargestellt ist. Dabei ist der logische Inhalt des Speicherelements 20 mit von oben nach unten ansteigender Speicheradresse dargestellt.

An einer ersten Speicheradresse, beispielsweise an der logischen Adresse $0000, ist ein erster Satz 30 von Konfigurationsdaten abgelegt. Ein zweiter Satz 40 von Konfigurationsdaten ist hinter dem ersten Satz 30 abgelegt, beispielsweise an der logischen Adresse $0100. Hinter dem zweiten Satz 40 von Konfigurationsdaten ist hier ein Abschlussdatenelement 50 abgelegt, beispielsweise an der logischen Adresse $0300.

Die logischen Adressen werden vorliegend zum besseren Verständnis in Hexadezimalzahlen ($...) angegeben, so entsprechen $0100 einem Wert von 256, $0200 einem Wert von 512, usw.

Der erste Satz 30 von Konfigurationsdaten kann für eine ältere Generation von elektrochirurgischen Versorgungsgeräten 10 vorgesehen sein. Solche Versorgungsgeräte erwarten nur einen einzigen Satz von Konfigurationsdaten auf dem Speicherelement 20, welcher sich immer an der logischen Adresse $0000 befindet. Dieser Satz von Konfigurationsdaten ist in seinem Inhalt eingeschränkt, da er nur Parameter für elektrochirurgische Instrumente und Signalformen enthalten kann, die bei der Entwicklung der entsprechenden Generation von elektrochirurgischen Versorgungsgeräten bereits bekannt waren.

Für neuere Instrumente oder Signalformen ist der zweite Satz 40 von Konfigurationsdaten vorgesehen. Ein modernes elektrochirurgisches Versorgungsgerät 10 ist in der Lage, Konfigurationsdaten auch von anderen logischen Adressen des Speicherelements 20 zu lesen, und kann so auf den zweiten Satz 40 von Konfigurationsdaten zugreifen.

In dem zweiten Satz 40 von Konfigurationsdaten können Konfigurationsdaten hinterlegt sein, welche die Konfigurationsdaten des ersten Satzes 30 ergänzen, so dass sie nur mit diesen Konfigurationsdaten zusammen genutzt werden können. Alternativ können die im zweiten Satz 40 hinterlegten Konfigurationsdaten in sich vollständig sein.

Die logische Adresse, an welcher der zweite Satz 40 von Konfigurationsdaten abgelegt ist, hängt von der Länge des ersten Satzes 30 von Konfigurationsdaten ab. Dabei kann die Länge des ersten Satzes 30 fest und bekannt sein, so dass die logische Adresse des zweiten Satzes 40 ebenfalls bekannt ist.

Der erste Satz 30 von Konfigurationsdaten kann auch eine variable Länge aufweisen. In diesem Fall umfasst der erste Satz 30 ein erstes Längendatenelement 31, welches die Länge das ersten Satzes 30 und/oder die Adresse des nächsten Satzes von Konfigurationsdaten anzeigt.

Der zweite Satz 40 von Konfigurationsdaten wird in der Regel immer eine variable Länge aufweisen und daher ebenfalls ein zweites Längendatenelement 41 umfassen, welches die Länge des zweiten Satzes 40 anzeigt.

Neben dem ersten Satz 30 und dem zweiten Satz 40 können beliebig viele weitere Sätze von Konfigurationsdaten in dem Speicherelement 20 abgelegt sein. Um das Ende der Datensätze anzuzeigen, ist hinter dem letzten Satz das Abschlussdatenelement 50 abgelegt.

Die einzelnen Sätze 30, 40 können direkt aneinander anschließen. In der Regel wird jedoch das Speicherelement 20 nur blockweise gelesen und/oder beschrieben werden können, beispielsweise in Blöcken mit einer Länge von $0100. Da die einzelnen Sätze 30, 40 nicht zwangsläufig ebenfalls eine Länge von $0100 haben, können zwischen einzelnen Sätzen 30, 40 bzw. dem Abschlussdatenelement 50 ungenutzte Speicherbereiche liegen.

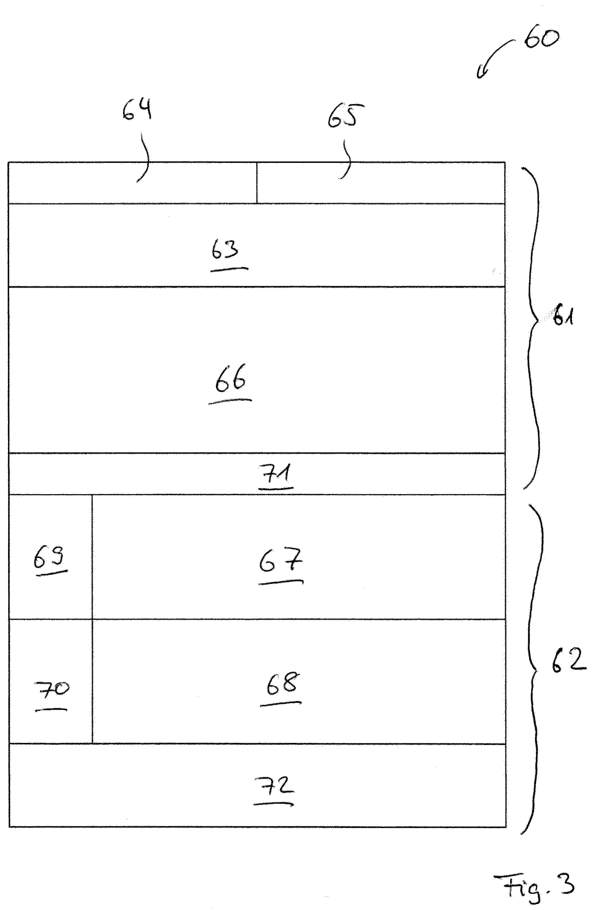

In Figur 3 ist ein möglicher Aufbau eines Satzes 60 von Konfigurationsdaten im Detail dargestellt, welcher anstelle von oder zusätzlich zu den Sätzen 30, 40 in der Datenstruktur auf dem Speicherelement 20 abgelegt sein kann.

Der Satz 60 weist zwei Abschnitte 61, 62 auf.

Der erste Abschnitt 61 beginnt mit einem Definitionsdatenelement 63.

Das Definitionsdatenelement 63 enthält Angaben zu der Struktur des Satzes 60, d.h. Typen- und/oder Versionsbeschreibung, Längen- und/oder Postionsangaben dieses und weiterer Abschnitte innerhalb des Satzes 60, und/oder Positionsangaben des nächsten Satzes von Konfigurationsdaten, sowie spezifische Informationen des Speicherelements 20, wie beispielsweise die Blockgröße, mit der auf dem Speicherelement gelesen oder geschrieben werden kann. Dabei können Typen- und/oder Versionsdaten in einem Typdatenelement 64 enthalten sein, und Längen- und/oder Positionsangaben können in einem Längendatenelement 65 enthalten sein. Das Typdatenelement 64 und/oder das Längendatenelement 65 können eigenständige Datenelemente oder Unterelemente des Definitionsdatenelements 63 sein.

Mit Hilfe des Definitionsdatenelements 63 ist das Versorgungsgerät 10 imstande zu entscheiden, ob es mit dem Datensatz 60 kompatibel ist.

Falls der Satz 60 der letzte Satz Konfigurationsdaten in der Datenstruktur ist, kann als logische Speicheradresse des nächsten Satzes $0000 in dem Definitionsdatenelement 63 eingetragen sein.

Das Definitionsdatenelement 63 entscheidet über die Struktur des Parameterdatenelements 66 sowie des weiteren Abschnitts 62.

Das Parameterdatenelement 66 enthält die eigentlichen Konfigurationsdaten zur Bestimmung des vom Versorgungsgerät 10 abzugebenden elektrischen Signals.

Der Abschnitt 61 des Satzes 60 von Konfigurationsdaten ist als "read only" Bereich definiert, d.h. dass die in dem Abschnitt 61 hinterlegten Daten betriebsmäßig nicht durch das Versorgungsgerät 10 verändert werden können.

Der zweite Abschnitt 62 umfasst zwei Betriebsdatenelemente 67, 68. In den Betriebsdatenelementen 67, 68 werden durch das elektrochirurgische Versorgungsgerät 10 Betriebsdaten des elektrochirurgischen Instruments 11 abgelegt. Bei diesen Betriebsdaten kann es sich um Verbrauchsdaten, beispielsweise Anzahl und Dauer der Aktivierungen und/oder abgegebene Energie/Leistung des Instruments 11, Zeitstempel, Temperaturdaten oder ähnliches handeln. Die Betriebsdaten können durch das elektrochirurgische Versorgungsgerät 10 ausgewertet werden, um festzustellen, ob eine weitere Nutzung des Instruments 11 zulässig ist.

Um ein Beschreiben der Betriebsdatenelemente 67, 68 durch das Versorgungsgerät 10 zu ermöglichen, ist der Abschnitt 62 als "read / write" Bereich definiert, d.h. dass Schreibzugriffe durch das Versorgungsgerät 10 zulässig sind.

Da bei dem Beschreiben der Betriebsdatenelemente 67, 68 Fehler auftreten können, beispielsweise durch Störsignale, werden die Betriebsdatenelemente 67, 68 durch das Versorgungsgerät 10 abwechselnd beschrieben. Auf diese Weise ist sichergestellt, dass jeweils die im vorigen Schreibzugriff abgelegten Betriebsdaten noch verfügbar sind, wenn ein Schreibzugriff fehlgeschlagen ist.

Um zu ermitteln, welches der Betriebsdatenelemente 67, 68 die aktuellsten Betriebsdaten enthält, und welches als nächstes zu beschreiben ist, ist jedem der Betriebsdatenelemente 67, 68 ein Schreibflag zugeordnet 69, 70. Nach einem erfolgreichen Schreibvorgang in eines der Betriebsdatenelemente 67, 68 wird das zugeordnete Schreibflag 69 bzw. 70 getoggelt, also von "Null" auf "Eins" bzw. von "Eins" auf "Null" gesetzt.

Das Versorgungsgerät 10 liest vor jedem Lese- oder Schreibzugriff auf die Betriebsdatenelemente 67, 68 die Schreibflags 69, 70 aus. Haben beide Schreibflags 69, 70 den gleichen Wert, so sind die Betriebsdaten im Betriebsdatenelement 67 aktuell, und die nächsten Betriebsdaten sind in das Betriebsdatenelement 68 zu schreiben. Haben hingegen die Schreibflags 69, 70 unterschiedliche Werte, so sind die Betriebsdaten in dem Betriebsdatenelement 68 aktuell, und die nächsten Betriebsdaten sind in das Betriebsdatenelement 67 zu schreiben.

Im Auslieferungszustand des Instruments 11 sind die Abschnitte 67, 68, 69 und 70 mit einer vorgegebenen Zahlenfolge, beispielsweise der Fibonacci-Folge, beschrieben. Das Versorgungsgerät 10 versucht zunächst diese Zahlenfolge zu erkennen. Enthalten diese Abschnitte diese Folge, weiß das Versorgungsgerät 10, dass es sich bei dem Instrument 11 um ein unbenutztes Instrument handelt.

Nach der ersten Nutzung des Instruments 11 schreibt das Versorgungsgerät Betriebsdaten in das Betriebsdatenelement 68, und setzt das Schreibflag 70 auf den Wert "Null". Bei den Betriebsdaten kann es sich um Verbrauchsdaten, beispielsweise Anzahl und Dauer der Aktivierungen und/oder abgegebene Energie/Leistung des Instruments 11, Zeitstempel, Temperaturdaten oder ähnliches handeln.

Bei der nächsten Benutzung des Instruments 11 mit dem Versorgungsgerät 10 oder mit einem anderen Versorgungsgerät, wird nun erkannt, dass der Abschnitt 62 nur die erste Hälfte der vorgegebenen Zahlenfolge, beispielsweise der Fibonacci-Folge, enthält. Dadurch weiß das Versorgungsgerät 10, dass die aktuellen Betriebsdaten im Betriebsdatenelement 68 abgelegt sind, und dass die nächsten Betriebsdaten in das Betriebsdatenelement 67 zu schreiben sind. Das Schreibflag 69 wird auf den Wert "Null" gesetzt.

Bei der nächsten Benutzung des Instruments 11 mit dem Versorgungsgerät 10, oder mit einem anderen Versorgungsgerät, werden die Flags 69, 70 ausgelesen. Da deren Werte nun gleich sind, weiß das Versorgungsgerät, dass die aktuellen Betriebsdaten im Betriebsdatenelement 67 abgelegt sind, und dass die nächsten Betriebsdaten in das Betriebsdatenelement 68 zu schreiben sind. Das Schreibflag 70 wird auf den Wert "Eins" gesetzt. Bei der nächsten Benutzung des Instruments 11 mit dem Versorgungsgerät 10, oder mit einem anderen Versorgungsgerät, werden wiederum die Flags 69, 70 ausgelesen. Da deren Werte nun ungleich sind, weiß das Versorgungsgerät, dass die aktuellen Betriebsdaten im Betriebsdatenelement 68 abgelegt sind, und dass die nächsten Betriebsdaten in das Betriebsdatenelement 67 zu schreiben sind.

Anstelle von zwei Schreibflags 69, 70 kann auch ein einziges Schreibflag verwendet werden, welches nach jedem Schreibvorgang getoggelt wird. Der Wert des Schreibflags gibt dann an, welches Betriebsdatenelement aktuelle Betriebsdaten enthält, und in welches Betriebsdatenelement als nächstes geschrieben werden soll.

Die Definition einzelner Bereiche des Speicherelements 20 als "read only" bzw. "read / write" ist wiederum nur für einzelne Speicherblöcke mit einer vorgegebenen Größe möglich, die nicht zwangsläufig mit den Größen der Abschnitte 61, 62 übereinstimmt. Daher können am Ende der Abschnitte 61, 62 ungenutzte Speicherbereiche 71, 72 vorhanden sein. Ähnliche, nicht dargestellte ungenutzte Speicherelemente können zwischen den einzelnen Datenelementen angeordnet sein.

Um die Integrität der in den einzelnen Datenelementen abgelegten Daten sicherzustellen, kann die Datenstruktur nicht dargestellte Prüfsummenelemente enthalten.

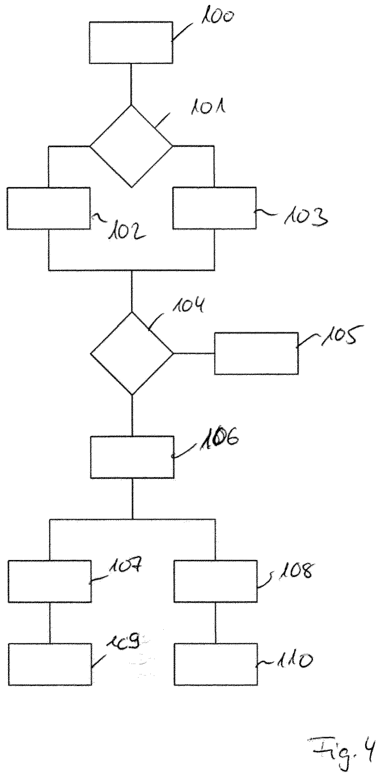

Ein Verfahren zum Lesen und Schreiben der Betriebsdaten durch ein Versorgungsgerät 10 ist in Figur 4 dargestellt. In einem ersten Schritt 100 werden die Schreibflags 69, 70 ausgelesen und in einem zweiten Schritt 101 miteinander verglichen. Haben beide Schreibflags 69, 70 den gleichen Wert, so wird in Schritt 102 das Betriebsdatenelement 67 ausgelesen. Sind die Werte der Schreibflags 69, 70 unterschiedlich, so wird in Schritt 103 das Betriebsdatenelement 68 ausgelesen.

In Schritt 104 wird anhand der ausgelesenen Betriebsdaten geprüft, ob eine weitere Nutzung des Instruments 11 zulässig ist. Ist dies nicht der Fall, so wird in Schritt 105 eine entsprechende Meldung durch das Versorgungsgerät 10 ausgegeben, und das Verfahren wird abgebrochen.

Ist die Nutzung zulässig, so wird das Instrument 11 in Schritt 106 durch das Versorgungsgerät 10 aktiviert, wobei aus dem Speicherelement 20 geladene Konfigurationsdaten und ggf. Benutzereingaben berücksichtigt werden.

Nach der Nutzung werden anhand des Ergebnisses des Vergleichs in Schritt 101 entweder in Schritt 107 aktuelle Betriebsdaten in das Betriebsdatenelement 68 geschrieben, wenn die Werte der Schreibflags 69, 70 gleich waren, oder in Schritt 108 aktuelle Betriebsdaten in das Betriebsdatenelement 67 geschrieben, wenn die Werte der Schreibflags 69, 70 unterschiedlich waren. Nach erfolgreichem Schreibvorgang wird das entsprechende Schreibflag 69 (Schritt 109) oder 70 (Schritt 110) getoggelt, damit ist das Verfahren abgeschlossen.

Für den Fall, dass die Datenstruktur mehrere Sätze von Konfigurationsdaten enthält, die beschreibbare Betriebsdatenelemente umfassen, so sollten aktuelle Betriebsdaten in jeden Satz einzeln geschrieben werden. Nur so kann sichergestellt sein, dass ein Versorgungsgerät, welches nur einen Teil der Sätze von Konfigurationsdaten ausliest, auch auf aktuelle Betriebsdaten zugreift.

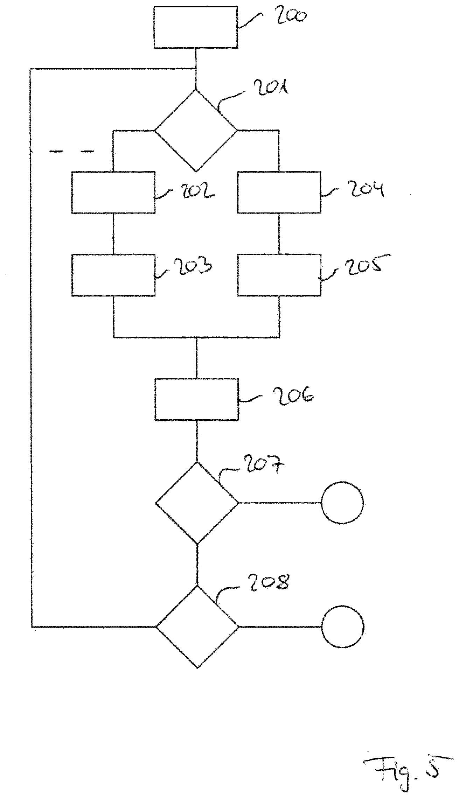

Ein Verfahren zum Auslesen der Sätze 30, 40, 60 von Betriebsdaten aus dem Speicherelement 20 durch das Versorgungsgerät 10 ist in Figur 5 dargestellt. Hierbei wird in einem ersten Schritt 200 ein erster Datenblock, beginnend bei der logischen Adresse $0000, aus dem Speicherelement ausgelesen. In Schritt 201 wird geprüft, ob der gelesene Datenblock ein Längendatenelement 31 eines ersten Satzes 30 von Konfigurationsdaten enthält. Ist dies der Fall, so wird in Schritt 202 der komplette erste Satz 30 aus dem Speicher 20 ausgelesen, und in Schritt 203 wird die logische Speicheradresse des nächsten Satzes von Konfigurationsdaten anhand des Inhalts des Längendatenelements 31 bestimmt.

Enthält der erste Datenblock kein Längendatenelement, so wird in Schritt 204 der erste Satz 30 unter der Annahme gelesen, dass dieser eine feste bekannte Länge aufweist. Dementsprechend wird in Schritt 205 die logische Speicheradresse des nächsten Satzes von Konfigurationsdaten anhand der bekannten festen Länge des ersten Satzes 30 bestimmt.

Als nächstes wird in einem Schritt 206 ein Datenblock von der zuvor ermittelten nächsten logischen Speicheradresse gelesen, und in Schritt 207 wird geprüft, ob dieser Datenblock sinnvolle Daten enthält. Enthält der entsprechende Datenblock keine sinnvollen Daten, so sind alle Sätze aus der Speicherelement 20 ausgelesen und das Verfahren wird beendet.

Im vorliegenden Beispiel kann unter der Formulierung "enthält keine sinnvollen Daten" auch ein Fall enthalten sein, dass die ermittelte logische Speicheradresse außerhalb des zugreifbaren Speicherbereichs des Speicherelements 20 liegt, beispielsweise wenn der erste Satz 30 von Konfigurationsdaten in einem elektrochirurgischen Instrument einer älteren Generation das Speicherelement fast vollständig ausfüllt. In diesem Fall muss die Software zur Umsetzung des beschriebenen Verfahrens in der Lage sein, einen ggf. auftretenden Speicheradressfehler abzufangen.

Im Übrigen umfasst die Formulierung "enthält keine sinnvollen Daten" jeden Fall, in dem der gelesene Datenblock nicht entweder ein Typdatenelement und/oder ein Längendatenelement eines weiteren Satzes von Konfigurationsdaten, oder ein Datenabschlusselement umfasst.

In einem nächsten Schritt 208 wird dann geprüft, ob der gelesene Datenblock ein Datenabschlusselement 50 beinhaltet. In diesem Fall sind ebenfalls alle Sätze von Konfigurationsdaten ausgelesen, und das Verfahren wird beendet.

Enthält der Datenblock hingegen ein Typdatenelement und/oder ein Längendatenelement eines weiteren Satzes von Konfigurationsdaten, so wird das Verfahren ab Schritt 201 wiederholt. Die Abfrage in Schritt 201 muss in die Schleife nicht einbezogen werden, wenn bis auf den ersten Satz 30 alle weiteren Sätze von Konfigurationsdaten immer ein Längendatenelement beinhalten. In diesem Fall kann nach Schritt 208 direkt zu Schritt 202 gesprungen werden, was durch die gestrichelte Linie in Figur 5 angedeutet ist.

Nach Abschluss des Verfahrens kann das Versorgungsgerät 10 anhand der Inhalte der eingelesenen Typdatenelemente ermitteln, ob die jeweiligen Sätze mit dem Versorgungsgerät 10 kompatibel sind, und nur solche kompatiblen Sätze berücksichtigen.

## Patentansprüche

1. Elektrochirurgisches System, umfassend:
- ein elektrochirurgisches Versorgungsgerät (10), und
- ein elektrochirurgisches Instrument (11), wobei
das elektrochirurgische Instrument (11) ein Speicherelement (20) umfasst, welches durch das elektrochirurgische Versorgungsgerät (10) ausgelesen und beschrieben werden kann, wenn das elektrochirurgische Instrument (11) mit dem elektrochirurgischen Versorgungsgerät (10) verbunden ist, wobei das elektrochirurgische Versorgungsgerät eingerichtet ist, Betriebsdaten auf das Speicherelement (20) zu schreiben,
**dadurch gekennzeichnet, dass** die Betriebsdaten auf dem Speicherelement in einer Datenstruktur abgelegt sind, welche ein erstes Betriebsdatenelement (67) und ein zweites Betriebsdatenelement (68) umfasst, und
dass das elektrochirurgische Versorgungsgerät (10) eingerichtet ist, Betriebsdaten abwechselnd in das erste Betriebsdatenelement (67) oder das zweite Betriebsdatenelement (68) zu schreiben.

2. Elektrochirurgisches System nach Anspruch 1, **dadurch gekennzeichnet, dass** die Datenstruktur ein Schreibflag (69, 70) umfasst, dessen Inhalt anzeigt, ob Betriebsdaten in das erste Betriebsdatenelement (67) oder das zweite Betriebsdatenelement (68) geschrieben werden sollen.

3. Elektrochirurgisches System nach Anspruch 2, **dadurch gekennzeichnet, dass** das elektrochirurgische Versorgungsgerät (10) eingerichtet ist, nach dem erfolgreichen Schreiben von Betriebsdaten in das erste Betriebsdatenelement (67) oder das zweite Betriebsdatenelement (68) das Schreibflag (69, 70) zu toggeln.

4. Elektrochirurgisches System nach Anspruch 2 oder 3, **dadurch gekennzeichnet, dass** in der Datenstruktur jedem der Betriebsdatenelemente (67, 68) ein Schreibflag (69, 70) zugeordnet ist, und dass das elektrochirurgische Versorgungsgerät (10) eingerichtet ist, nach dem erfolgreichen Schreiben von Betriebsdaten in eines der Betriebsdatenelemente (67, 68) das diesem Betriebsdatenelement zugeordnete Schreibflag zu toggeln.

5. Elektrochirurgisches System nach Anspruch 4, **dadurch gekennzeichnet, dass** das elektrochirurgische Versorgungsgerät (10) eingerichtet ist, Betriebsdaten in das erste Betriebsdatenelement (67) zu schreiben, wenn der Wert der Schreibflags (69, 70) ungleich ist, und Betriebsdaten in das zweite Betriebsdatenelement (68) zu schreiben, wenn der Wert der Schreibflags (69, 70) gleich ist.

6. Verfahren zum Schreiben von Betriebsdaten auf ein Speicherelement (20) eines elektrochirurgischen Instruments (11) in einem elektrochirurgischen System (1) gemäß eines der Ansprüche 2 bis 5, mit den Schritten:
a) Auslesen des Werts des Schreibflags aus der Datenstruktur, die auf dem Speicherelement (20) abgelegt ist,
b) Ermitteln, anhand des Wertes des Schreibflags, ob Betriebsdaten in das erste Betriebsdatenelement oder in das zweite Betriebsdatenelement geschrieben werden sollen,
c) Schreiben der Betriebsdaten in das ermittelte Betriebsdatenelement, und
d) Toggeln des Schreibflags.

7. Verfahren nach Anspruch 6, wobei die Werte von zwei Schreibflags ausgelesen werden, welche jeweils einem Betriebsdatenelement zugeordnet sind, und wobei die Betriebsdaten in das erste Betriebsdatenelement geschrieben werden, wenn die Werte der beiden Schreibflags ungleich sind, und in das zweite Betriebsdatenelement geschrieben werden, wenn die Werte der beiden Schreibflags gleich sind.

8. Verfahren nach Anspruch 7, **dadurch gekennzeichnet, dass** nach dem Schreiben von Betriebsdaten in eines der Betriebsdatenelemente das diesem Betriebsdatenelement zugeordnete Schreibflag getoggelt wird.

## Claims

1. Electrosurgical system comprising:
- an electrosurgical supply device (10), and
- an electrosurgical instrument (11), wherein
the electrosurgical instrument (11) comprises a memory element (20) which can be read out and written to by the electrosurgical supply device (10) when the electrosurgical instrument (11) is connected to the electrosurgical supply device (10), wherein
the electrosurgical supply device is configured to write operating data to the memory element (20),
**characterized in that** the operating data are stored on the storage element in a data structure which comprises a first operating data element (67) and a second operating data element (68), and
**in that** the electrosurgical supply device (10) is configured to write operating data alternately to the first operating data element (67) or the second operating data element (68).

2. Electrosurgical system according to claim 1, **characterized in that** the data structure comprises a write flag (69, 70), the content of which indicates whether operating data is to be written to the first operating data element (67) or the second operating data element (68).

3. Electrosurgical system according to claim 2, **characterized in that** the electrosurgical supply device (10) is configured to toggle the write flag (69, 70) after the successful writing of operating data to the first operating data element (67) or the second operating data element (68).

4. Electrosurgical system according to claim 2 or 3, **characterized in that** a write flag (69, 70) is assigned to each of the operating data elements (67, 68) in the data structure, and **in that** the electrosurgical supply device (10) is configured to toggle the write flag assigned to this operating data element after the successful writing of operating data to one of the operating data elements (67, 68).

5. Electrosurgical system according to claim 4, **characterized in that** the electrosurgical supply device (10) is configured to write operating data into the first operating data element (67) if the value of the write flags (69, 70) is unequal, and to write operating data into the second operating data element (68) if the value of the write flags (69, 70) is equal.

6. Method for writing operational data to a memory element (20) of an electrosurgical instrument (11) in an electrosurgical system (1) according to any one of claims 2 to 5, comprising the steps of:
a) reading the value of the write flag from the data structure stored on the memory element (20),
b) determining, on the basis of the value of the write flag, whether operating data is to be written to the first operating data element or to the second operating data element,
c) writing the operating data to the determined operating data element, and
d) toggling the write flag.

7. Method according to claim 6, wherein the values of two write flags are read out, which are each assigned to an operating data element, and wherein the operating data are written into the first operating data element if the values of the two write flags are not equal, and are written into the second operating data element if the values of the two write flags are equal.

8. Method according to claim 7, **characterized in that** after writing operating data into one of the operating data elements, the write flag assigned to this operating data element is toggled.

## Revendications

1. Système électrochirurgical, comprenant :
- un dispositif d'alimentation électrochirurgical (10), et
- un instrument électrochirurgical (11), dans lequel
l'instrument électrochirurgical (11) comprend un élément de mémoire (20) qui peut être lu et écrit par le dispositif d'alimentation électrochirurgical (10) lorsque l'instrument électrochirurgical (11) est connecté au dispositif d'alimentation électrochirurgical (10), dans lequel
le dispositif d'alimentation électrochirurgical est configuré pour écrire des données de fonctionnement sur l'élément de mémoire (20),
**caractérisé en ce que** les données de fonctionnement sont enregistrées sur l'élément de mémoire dans une structure de données qui comprend un premier élément de données de fonctionnement (67) et un deuxième élément de données de fonctionnement (68), et
**en ce que** le dispositif d'alimentation électrochirurgical (10) est configuré pour écrire des données de fonctionnement alternativement dans le premier élément de données de fonctionnement (67) ou dans le deuxième élément de données de fonctionnement (68).

2. Système électrochirurgical selon la revendication 1, **caractérisé en ce que** la structure de données comprend un indicateur d'écriture (69, 70) dont le contenu indique si les données de fonctionnement doivent être écrites dans le premier élément de données de fonctionnement (67) ou dans le deuxième élément de données de fonctionnement (68).

3. Système électrochirurgical selon la revendication 2, **caractérisé en ce que** l'appareil d'alimentation électrochirurgical (10) est configuré pour basculer l'indicateur d'écriture (69, 70) après l'écriture réussie de données de fonctionnement dans le premier élément de données de fonctionnement (67) ou le deuxième élément de données de fonctionnement (68).

4. Système électrochirurgical selon la revendication 2 ou 3, **caractérisé en ce que** dans la structure de données, un indicateur d'écriture (69, 70) est associé à chacun des éléments de données de fonctionnement (67, 68), et **en ce que** le dispositif d'alimentation électrochirurgical (10) est configuré, après l'écriture réussie de données de fonctionnement dans l'un des éléments de données de fonctionnement (67, 68), pour basculer l'indicateur d'écriture associé à cet élément de données de fonctionnement.

5. Système électrochirurgical selon la revendication 4, **caractérisé en ce que** le dispositif d'alimentation électrochirurgical (10) est configuré pour écrire des données de fonctionnement dans le premier élément de données de fonctionnement (67) lorsque la valeur des indicateurs d'écriture (69, 70) n'est pas égale, et pour écrire des données de fonctionnement dans le deuxième élément de données de fonctionnement (68) lorsque la valeur des indicateurs d'écriture (69, 70) est égale.

6. Procédé d'écriture de données de fonctionnement sur un élément de mémoire (20) d'un instrument électrochirurgical (11) dans un système électrochirurgical (1) selon l'une des revendications 2 à 5, comprenant les étapes de :
a) lire la valeur de l'indicateur d'écriture dans la structure de données enregistrée sur l'élément de mémoire (20),
b) déterminer, à l'aide de la valeur du drapeau d'écriture, si les données de fonctionnement doivent être écrites dans le premier élément de données de fonctionnement ou dans le deuxième élément de données de fonctionnement,
c) écrire les données de fonctionnement dans l'élément de données d'exploitation déterminé, et
d) basculer l'indicateur d'écriture.

7. Procédé selon la revendication 6, dans lequel les valeurs de deux indicateurs d'écriture sont lues, lesquelles sont respectivement associées à un élément de données de fonctionnement, et dans lequel les données de fonctionnement sont écrites dans le premier élément de données de fonctionnement lorsque les valeurs des deux indicateurs d'écriture ne sont pas égales, et sont écrites dans le deuxième élément de données de fonctionnement lorsque les valeurs des deux indicateurs d'écriture sont égales.

8. Procédé selon la revendication 7, **caractérisé en ce qu'**après l'écriture de données de fonctionnement dans l'un des éléments de données de fonctionnement, l'indicateur d'écriture associé à cet élément de données de fonctionnement est basculé.
